# EUROPEAN PATENT SPECIFICATION

(11) **EP 2 421 695 B1**
(45) Date of publication and mention of the grant of the patent: **12.02.2014**
(21) Application number: 10767764.3
(22) Date of filing: 22.04.2010
(51) Int. Cl.: B32B 3/12

(54) **SMART ENZYME-CONTAINING CATALYTIC PEELABLE COATINGS**
INTELLIGENTE ENZYMHALTIGE KATALYTISCHE ABLÖSBARE BESCHICHTUNGEN
REVÊTEMENTS CATALYTIQUES PELABLES INTELLIGENTS CONTENANT UNE ENZYME

(30) Priority: 22.04.2009 US 171631 P
(43) Date of publication of application: 29.02.2012
(73) Proprietor: ICX-Agentase, Pittsburgh, Pennsylvania 15238 (US)
(72) Inventor: WALKER, Jeremy P., Oakmont, Pennsylvania 15139 (US); GIDEL, Jonita Glenna, Pittsburgh, Pennsylvania 15217 (US)
(74) Representative: Grünecker, Kinkeldey, Stockmair & Schwanhäusser
(86) International application number: PCT/US2010/032047
(87) International publication number: WO 2010/124084

(56) References cited:
- WO-A1-2008/141436
- US-A1- 2004 109 853
- US-A1- 2007 014 838
- US-A1- 2009 023 881
- US-B2- 6 762 213
- DREVON G F ET AL: "High-activity enzyme-polyurethane coatings", BIOTECHNOLOGY AND BIOENGINEERING, WILEY & SONS, HOBOKEN, NJ, US, vol. 79, no. 7, 30 September 2002 (2002-09-30), pages 785-794, XP002257133, ISSN: 0006-3592, DOI: 10.1002/BIT.10334
- GUNDUZ ET AL: "BADGES OF IMMOBILIED ENZYMES: DETECTION OF CHEMICAL WARFARE AGENTS", , 1 July 2003 (2003-07-01), XP002685130, Retrieved from the Internet: URL:http://oai.dtic.mil/oai/oai?verb=getRe cord&metadataPrefix=html&identifier=ADA482 763 [retrieved on 2012-10-08]

## Description

### Field of the Invention

The present invention relates to a coating material comprising enzymes and pH-responsive elements incorporated into a strippable coating that recognizes, mitigates, detoxifies, and discloses hazardous chemicals such as toxic industrial chemicals, chemical toxins, and chemical warfare agents (known and emerging threats) and their simulants (hereinafter, "toxic chemicals"). Upon exposure to these toxic chemicals, the enzyme/coating material will immediately begin to detoxify the threat, reducing the contact hazard associated with the surface. The coating material of the invention simultaneously undergoes a color change that indicates to an observer a hazardous environment as well as identifying the exact location of contamination. Additionally, the coating is peelable and is highly absorptive of toxic chemicals, for example, chemical warfare agents and their simulants, and enable the war fighter to focus precise decontamination efforts.

### Background of the Invention

Biocatalysts (enzymes) are proteins that enable the rapid chemical transformations of organic compounds. Enzymes are powerful catalysts because they are highly specific, operate at ambient temperatures and are environmentally friendly. Several enzymes are capable of decontaminating toxic chemicals by breaking them down into benign or less harmful materials. Hydrolases are enzymes that utilize water to catalyze the hydrolysis of a chemical bond. Several hydrolase enzymes have been shown to facilitate the detection and decontamination of blister and organophosphate nerve chemical warfare agents. When these enzymes hydrolyze nerve and blister agents, acid is produced as a hydrolysis product. Because of the acid production, a pH-sensitive dye can provide an indication of the reaction. More preferably, specific pH-sensitive dyes can be used which generate visible colorimetric signals that are visually apparent to the observer under standard ambient lighting conditions. While enzymes are successfully employed in several different applications, many have practical limitations that minimize their functional utility. Enzymes typically have short shelf and operational lifetimes as they are complex, three dimensional biomolecules that rely heavily on specific conformation for their catalytic activity. U.S. Patent No. 6,762,213 discloses a polymer including at least one enzyme that is selected to catalyze a reaction of a substrate. The enzyme is immobilized within the polymer. The present invention provides a method of incorporating enzymes as co-monomers during polymer synthesis that result in a biocomposite material that has stable catalytic activity and that is resistant to many environmental stimuli that often negatively impact enzyme activity. The resulting material is a porous foam that contains covalently immobilized enzyme capable of catalyzing its substrate and producing a change in the state of the system which can be detected by an additional dye. These sensors' utility is limited to a single, brief use.

Chemical agents are defined as chemicals intended to kill or seriously injure human beings. Common examples of chemical agents include: G-series nerve agents, V-series nerve agents and blister agents. A wide variety of equipment designed to detect, identify and decontaminate chemical agents is currently in use by military and civilian forces. Many challenges exist with current technologies such as speed, accuracy, sensitivity, compatibility and logistical burden. Rapid, precise detection is essential so that users can recognize a threat, don the appropriate protective gear, and mitigate the hazard. Some examples of state-of-the-art chemical agent detection include: M8 paper, M256A1 kit, M272 kit, colorimetric tubes (Dräger tubes), electronic monitoring devices (ICAM) and CAD-Kit sensors.

M8 paper is used to detect liquids and aerosols by changing color according to the type of agent. It cannot be used to test vapors for the presence of chemical warfare agents. M8 paper responds within 30 seconds of exposure by turning one of three colors. Yellow indicates the presence of a G-series nerve agent; green designates a V-series nerve agent; red specifies a blister agent. Although M8 paper is very sensitive, it lacks specificity and is prone to error because it reacts with non-hazardous contaminants such as antifreeze and petroleum products, resulting in false-positive readings.

The M256A1 chemical agent detection kit is designed to detect and identify chemical agents present either as liquid or as vapor and consists of a) a booklet of M8 paper to detect agents in liquid form and b) foil-wrapped detector tickets to detect chemical vapors. The vapor sampler uses wet chemistry technology in which ampoules containing different substrates are crushed so that the liquids interact with strips of filter paper, chromatographic media and glass fiber filter. These substrates are then exposed to the vapor under suspicion. The reaction causes a color change, alerting the user to the presence of chemical agent. Blood, blister and nerve agents can be detected in 15 minutes. During the test, the ticket must be kept out of direct sunlight and should be held stationary to reduce evaporation of the reagents therein. This device is very sensitive and can detect agents at levels below those that can kill or injure people; however, it is prone to false-positive results and contains potentially carcinogenic reagents.

The M272 chemical agent water testing kit is designed to test for Lewisite, nerve, cyanide, and mustard chemical agents which may be present in water. It requires 60 mL water sample and uses reagent tablets, color tubes and heating, which is provided by lighted matches. This kit is operative between 32 and 125 °F. Water samples below 50 °F require warming before analysis. Water that is too hot may cause foaming in the detector tubes and thus requires cooling to below 105 °F. Once an acceptable temperature is achieved, the full range of tests can be conducted in twenty minutes. Results are qualitative and indicate only the presence of contaminants, not an actual concentration thereof.

Dräger tubes are colorimetric devices that detect and identify chemical agents. Air samples are drawn into a specific detector tube using a hand pump and then the presence and concentration of a substance can be determined. A different tube must be used for each agent; efficient use of this system, therefore, demands knowledge of which chemical agent is likely to be present. A tube for each possible chemical agent must be used for thorough detection. To simplify this process, a chip measurement system analyzer integrates an optical system for analyzing the color reaction, a flow controller, a pump system and 10 capillaries, each capable of detecting a different agent. Ten agents can be detected and measured within 2 minutes using this device.

Improved Chemical Agent Monitor (ICAM) is a hand-held instrument that detects nerve, blood, and blister agents in the air. This device employs ion mobility spectroscopy wherein an air sample is drawn into a reaction chamber using a pump. The air molecules are then ionized and the ionized particles are subsequently passed through an electrical field toward an ion detector. Comparison of the mobilities of the different ionic species to electronically stored standards allows an onboard microcomputer to determine the type of agent and its relative concentration. The ICAM can only detect nerve and blister agents in vapor form. Furthermore, it is prone to erroneous detection in enclosed spaces and areas of strong vapor concentration (i.e. heavy smoke). It can also become saturated, requiring recalibration.

CAD-Kit pens are enzyme-based sensors that detect chemical agents and toxic industrial chemicals on surfaces and in liquid samples. These sensors produce colorimetric results within five minutes of sampling, exhibit detection limits that rival those of expensive handheld electronic devices, and are resistant to most chemical interference, thus reducing the incidence of false-positive detection events. CAD-Kit sensors are highly specific in their detection; however, this specificity may necessitate the use of multiple sensors to identify an unknown chemical agent. A different pen must be used for detecting each chemical agent.

Many decontaminant formulations for chemical agents comprise harsh solvents and oxidizing agents. Aggressive solvents are used to solvate the chemical agent itself as well as any polymers that may have been added to them in order to thicken, and better weaponize the agent so that it persists in the environment. Many strong caustic materials such as sodium hydroxide, calcium hypochlorite, peroxides, and sodium hypochlorite have been used. The employment of these strong decontamination agents results in corrosion, stress cracking, and paint degradation. These formulations are environmentally unfriendly and often undesirable for decontaminating sensitive surfaces and equipment. Many of these strong decontamination chemicals have a short useful life and must be mixed immediately prior to use. Mechanical agitation and abrasion of the contaminated surface is required for effective decontamination with these formulations. Currently decontamination processes require a considerable investment of time and effort, present sizeable logistical burdens, and pose significant hazards to personnel and the environment. Moreover, the current state-of-the-art detection and decontamination methods are active processes, requiring the direct involvement of people at the site of chemical agent contamination. The current invention represents a paradigm shift from the present active detection and decontamination efforts to a passive solution which requires no specialized equipment and only minimal user involvement.

Strippable coatings have been utilized for many years as decorative and protective coverings in a wide variety of applications. US Patent No. 3,000,748 describes a thermoplastic coating composed of glyceride and cellulose ester that is smooth, strong, adherent, peelable and transparent that can be used to protect and preserve perishable foods. US Patent No. 3,442,833 discloses a coating comprising cellulose acetate butyrate and atactic propylene-ethylene block co-polymer that forms a clear, flexible water- and grease-resistant film. US Patent No. 6,232,392 discloses a water-borne, acrylic-polymer based temporary coating. This quick-drying, cold-water-washable coating protects paint spray booths from overspray and can also be used to shield decorative finishes, especially those of vehicles, from damage during transportation and assembly. US Patent No. 5,773,091 divulges a protective water-based wax barrier coating from which graffiti and other soiling can be removed by treatment with pyrrolidinone. As a non-sacrificial coating, it is not routinely stripped from an underlying substrate but rather it is cleaned and can be re-applied as it degrades over time. US Patent No. 5,716,667 describes a temporary, acrylic, water-resistant, environmentally-friendly protective coating. It is designed to be applied to and protect automobiles from damage by contact with contaminants and other objects. Once the coated reaches its desired destination, the coating can be released as a continuous sheet.

Strippable coatings have also been employed by the military to camouflage and protect equipment and vehicles. Temporary peelable camouflage coatings that can absorb chemical agent have been developed. US Patent No. 5,120,369 discloses a temporary, sacrificial strippable polymer coating that protects aircraft, ships and other equipment by absorbing and dissolving toxic chemical agents. The coating immobilizes the threat, reduces the rate of volatilization thereof and prevents breakthrough contamination of the underlying surfaces. US Patent No. 5,091,447 describes a temporary, easily removable, polymeric coating which isolates and confines a liquid hazardous material such as radionuclide-contaminated primary coolant water. This membrane serves as an impermeable barrier that prevents the spread, deposition or migration of contamination. Although these coatings can passively absorb or contain contamination and thus potentially reduce contact hazard and off-gassing, they do not detoxify the threat nor can they disclose the presence or location of chemical warfare agents.

The prior art also includes Biotechnology and Bioengineering, vol. 79, 2002, pages 785-794, a document in the field of biosensors, and relating to the use of enzymes immobilised in coatings for use in biocatalytic separation, filtration and such like. The prior art also includes WO2008141436 disclosing a paper having an immobilised enzyme, a thermophilic phosphotriesterase from Sulfolobus solfataricus. Upon encountering an organophosphorous pesticide such as paraoxon, hydrolytic cleavage results in the production of yellow p-nitrophenolate. The system thereby has use as a detoxifier of for example organophosphorous compounds as found in pesticides and chemical warfare agents.

A peelable coating, capable of recognizing the presence of a chemical warfare agent, responding to mitigate and detoxify the hazard and disclosing the presence and location of chemical agents to an observer would be highly desirable for military use. Furthermore, development of passive systems that require no power to recognize the agents will provide a reduced logistical burden to the war fighter. Peelable coatings are an attractive option because they afford the war fighter the opportunity to assess the hazard in the field and provide the option to remove the coating. They are also useful because chemical agents have a tendency to penetrate porous surfaces such as paints, plastics and concrete, thus they present a persistent contact hazard. Use of chemical agent absorbing peelable coatings minimizes this problem by protecting the coated substrate.

### Summary of the Invention

The present invention provides a coating material for absorbing a toxic chemical applied to a surface. The coating material comprises at least one polymer coating, one or more purified enzymes immobilized or entrapped in at least one polymer coating for protecting a surface from contamination by one or more toxic chemicals, one or more reporter indicators that are responsive to one or more decontamination products of one or more toxic chemicals producing changes in pH for detecting and disclosing the location of the toxic chemical, and one or more buffers for establishing and maintaining the pH of the coating in a range from between about 5 to 10. This coating material is removable from the surface by mechanical force applied to the surface. The mechanical force includes peeling the coating material from the surface to which it is applied by hand, or optionally by the use of water applied to the surface having the coating material. Optionally, the water for removing the peelable coating material may be supplied to the surface having the coating material of the present invention under pressure, such as for example, but not limited to the use of a pressure washer that is commercially available from any number of commercial outlets, such as Sears, Inc. Preferably, the coating material of the present invention includes wherein the reporter indicator is one or more pH-responsive dyes or a blend of pH-responsive dyes.

In another embodiment of the coating material of this invention, the enzyme or enzymes is/are selected from the group consisting of lyases, hydrolases, oxidoreductases, transferases, isomerases, and ligases, and combinations thereof. The coating material of this invention comprises wherein the enzyme, enzymes, or combinations thereof decontaminate the toxic chemical or chemicals. The toxic chemicals are for example chemical warfare agents selected from the group consisting of V-series organophosphate nerve agents, G-series organophosphate nerve agents, non-traditional agents, sulfur mustard blister agents, and nitrogen mustard blister agents.

Another embodiment of the coating material of the present invention includes wherein one or more of the purified enzymes are selected from the group consisting of one or more of organophosphorus acid anhydrolase (OPAA), organophosphorus hydrolase (OPH), dehalogenase (DHG), and diisopropylfluorophosphatase (DFPase).

Yet another embodiment of the coating material of the present invention provides wherein the polymer coating is selected from the group consisting of polyurethanes, polyacrylics, polysiloxanes, epoxies, polyacrylates, polyvinyls, polysilicone, fluorinated polymers, polypropylene, and polyethylene. The coating material absorbs fifty to one hundred percent of the toxic chemical upon contact with the coating within a period of one to ninety minutes.

Another embodiment of the coating material of the present invention includes wherein the reporter indicator is at least one or more pH-responsive dyes or dye blends which produce a colorimetric, fluorescent, or phosphorescent reaction in the presence of a toxic chemical. Preferably, the pH-responsive dye, dyes, or dye blend is a colorimetric dye.

In another embodiment of this invention, the coating material absorbs one or more toxic chemicals and sequesters one or more toxic chemicals from reaching the underlying surface to which the coating material is applied.

Another embodiment of this invention provides a method for absorbing, detoxifying, detecting, and disclosing the location of one or more toxic chemicals comprising applying a coating material to a surface wherein the coating material comprises one or more polymer coatings, one or more purified enzymes immobilized or entrapped in one or more polymer coatings, one or more reporter indicators that are responsive to one or more decontamination products of one or more toxic chemicals producing changes in pH, and one or more buffers for establishing and maintaining the pH of the polymer coating between a pH ranging from about 5 to 10, wherein the coating material is removable from the surface by mechanical force applied to the coating material, subjecting the coating material applied to the surface to at least one or more toxic chemicals, and effecting absorption of at least one or more of the toxic chemicals by the coating material for detoxifying at least one or more of the toxic chemicals by one or more of the enzymes and for detecting and disclosing the location of one or more of the toxic chemicals by at least one or more of the reporter indicators. In a preferred embodiment of this invention, the method of this invention as set forth herein includes removing the coating material containing at least one or more of the toxic chemicals by mechanical force. In a most preferred embodiment of this invention, the method as set forth herein includes wherein said mechanical force is selected from the group of peeling the coating material by hand, by the use of water, or by the use of water under pressure.

In yet another embodiment of the method of this invention as set forth herein, the method comprises retaining the activity of one or more of the enzymes for a time period ranging from at least one week to six months.

Another embodiment of this invention discloses the method as set forth herein including detoxifying a surface contaminated with levels of at least one toxic chemical ranging from 0.1 mg per square meter to 10 grams per square meter.

A further embodiment of this invention includes a coating material, as described herein, comprising wherein the polymer coating is selected from the group consisting of a water-borne coating and a solvent-borne coating.

A preferred embodiment of this invention includes a coating material for absorbing a toxic chemical applied to a surface comprising one or more polymer coatings, one or more purified enzymes immobilized or entrapped in the polymer coating for protecting a surface from contamination by one or more toxic chemicals, one or more reporter agents that are responsive to one or more decontamination products of one or more toxic chemicals producing changes in pH for detecting and disclosing the location of at least one or more toxic chemicals wherein a visual color change of the coating occurs and is observable to the naked eye, and one or more buffers for establishing and/or maintaining the pH of the coating between a pH from about 5 to 10, wherein the coating is removable from the surface by mechanical force applied to the surface.

### Brief Description of the Drawings

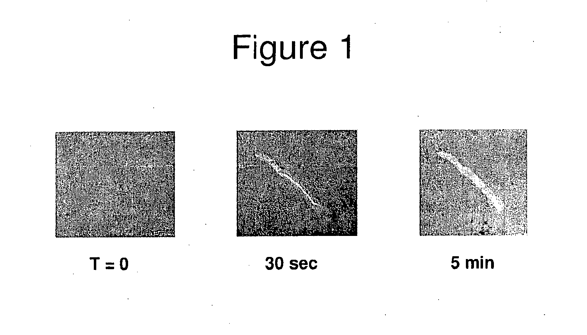

Figure 1 illustrates the disclosure by the present invention of applied DFP (diisopropylfluorophosphate), a G-series nerve agent simulant at various time periods after surface contamination.

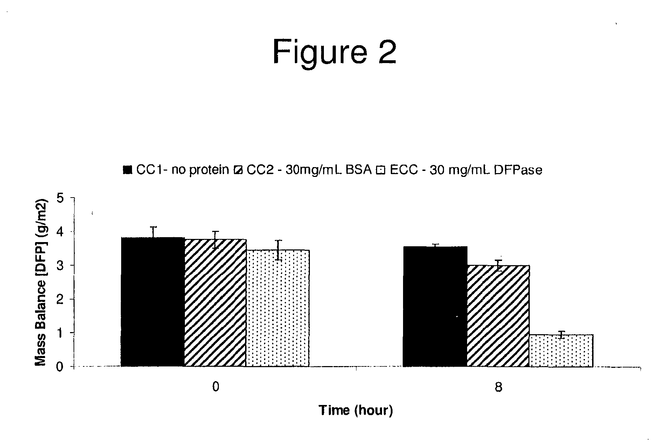

Figure 2 shows the DFP decontamination efficacy of the present invention (set forth as "ECC"(enzyme containing coating), versus two control coatings: CM1 (coating with no enzyme) and CC2 (coating containing BSA [bovine serum albumin], a non-enzymatic protein).

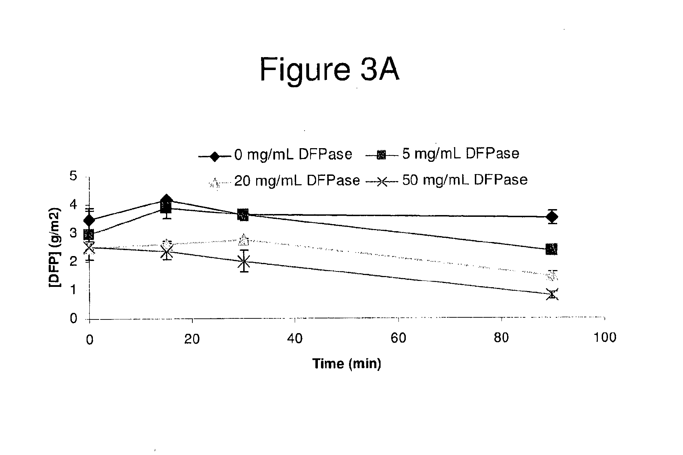

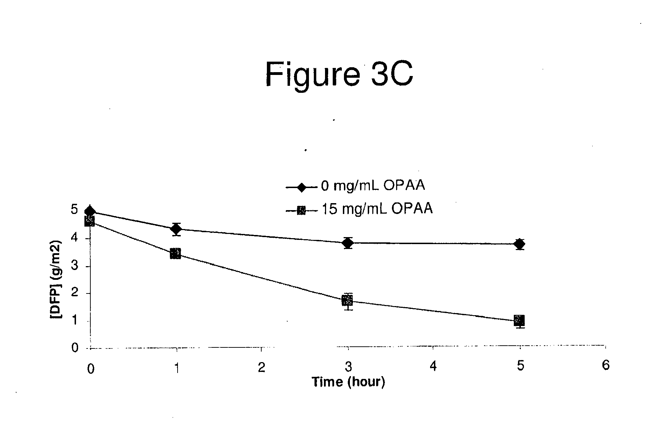

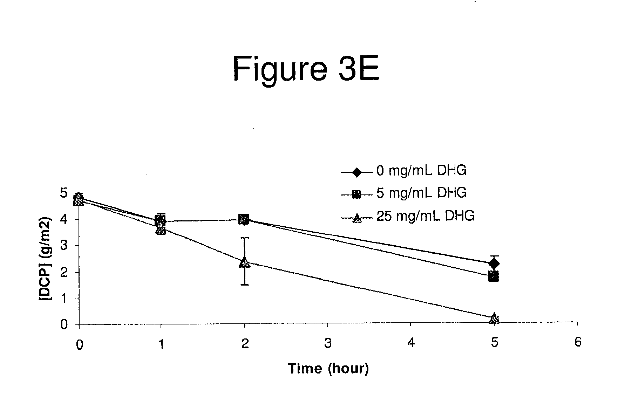

Figures 3A -B show the decontamination response of the coating material of the present invention having varying enzyme loading concentrations of DFPase when the coating material is challenged with DFP, a G-series nerve simulant. Figures 3C-D show the decontamination response of the coating material of the present invention having varying enzyme loading concentrations of OPAA when the coating material is challenged with DFP, a G-series nerve simulant. Figures 3E-F show the decontamination response of the coating material of the present invention having varying enzyme loading concentrations of DHG (dehalogenase) when the coating material is challenged with DCP (dichloropentane), a sulfur mustard simulant.

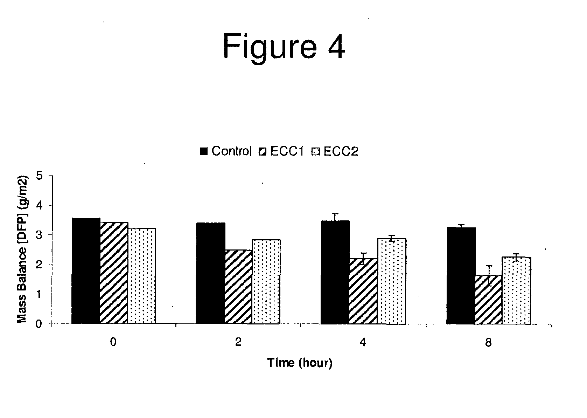

Figure 4 demonstrates the dependence on buffer concentration of the coating material of the present invention for decontamination of a surface contaminated with DFP over a time intervals of 0, 2, 4, and 8 hours. Control coating has no enzyme and 150mM BTP at pH 9; "ECC1" is a coating material of the present invention having 30mg/ml DFPase and 150mM BTP buffer at pH 9; and ECC2 is a coating material of the present invention with 30mg/ml DFPase and 75mM BTP buffer at pH 9.

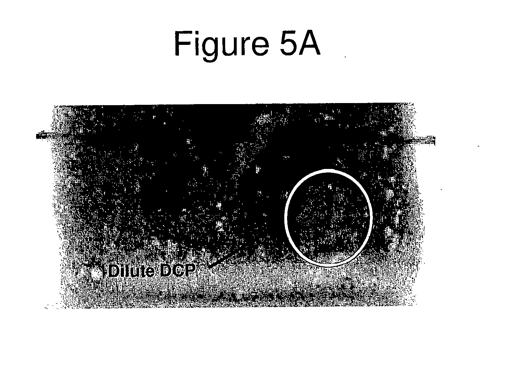

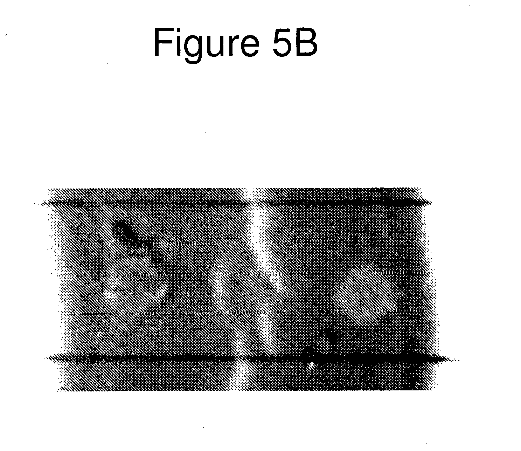

Figures 5A and B depict the disclosure by the present invention of applied DCP, a sulfur mustard analog (Figure 5A-neat DCP on left and dilute DCP on right, with coating material having 10mg/ml DHG and 15mMBTP buffer at pH 9, and Figure 5B-neat DCP on left side and dilute DCP on right side, with coating material having 10mg/ml DGH and 1.5mM BTP buffer at pH 9).

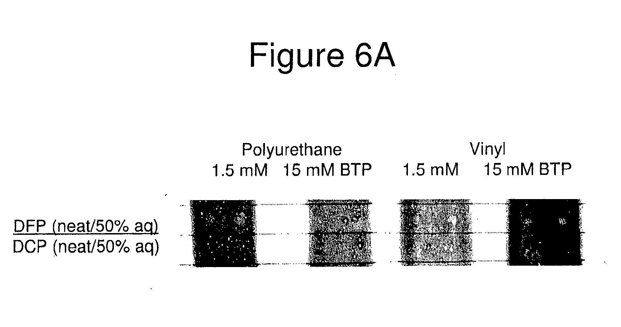

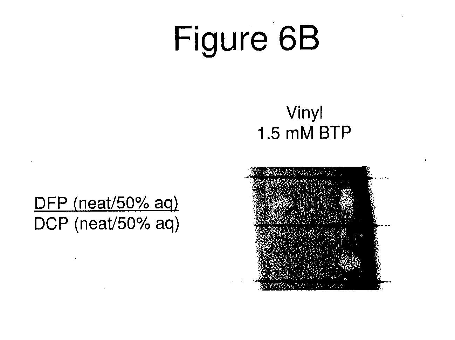

Figure 6A shows broad spectrum disclosure (detection) of multiple simulants DFP and DCP by coating material of the present invention that contain both 5mg/ml DFPase and 5mg/ml DHG enzymes in polymer coatings of polyurethane or vinyl, and either 1.5mM or 15mMBTP buffer, after a 60 minute time lapse. Figure 6B shows broad spectrum disclosure (detection) of multiple simulants DFP and DCP by coating material of the present invention that contain both 2mg/ml DFPase and 10mg/ml DHG enzymes in vinyl polymer coatings and 1.5mM BTP buffer, after a 60 minute time lapse.

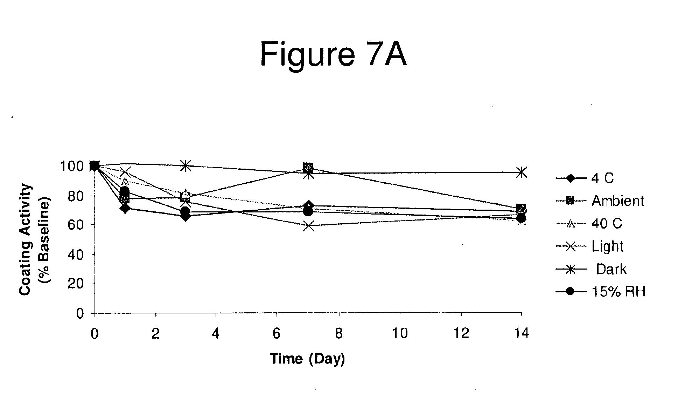

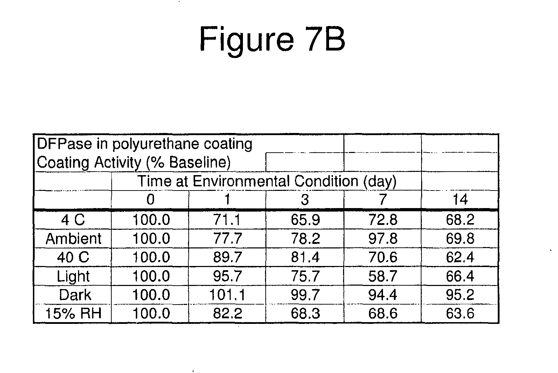

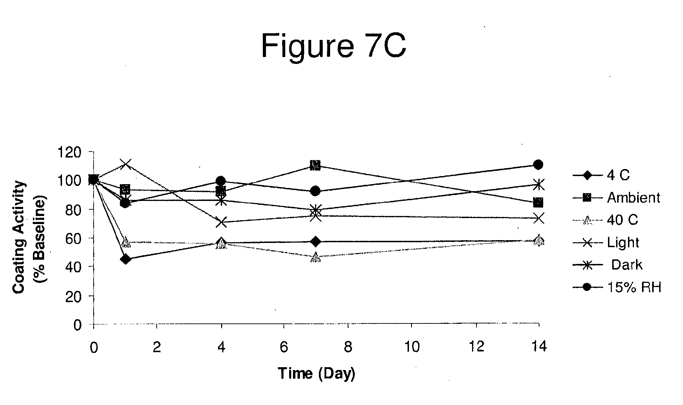

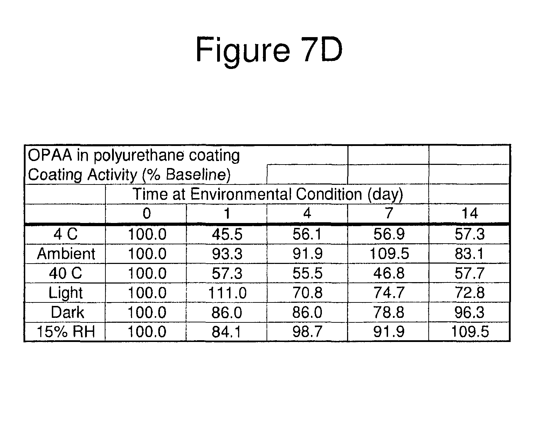

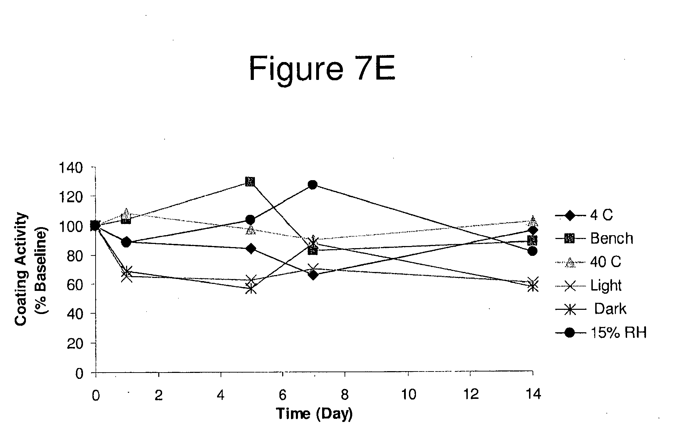

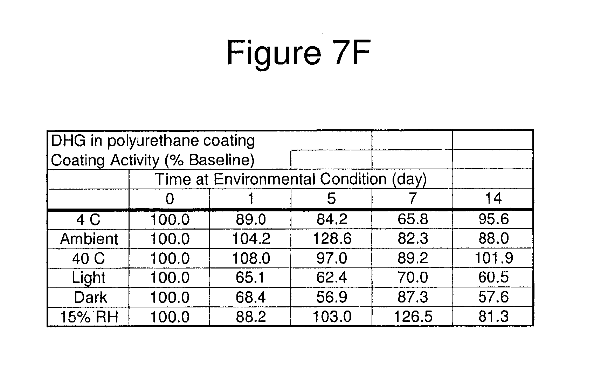

Figures 7A and 7B show the stability of the coating material of the present invention having a DFPase enzyme in a polyurethane coating after storage in several different environmental conditions over the period of 14 days. Figures 7C and 7D show the stability of the coating material of the present invention having a OPAA enzyme in a polyurethane coating after storage in several different environmental conditions over the period of 14 days. Figures 7E and 7F show the stability of the coating material of the present invention having a DHG enzyme in a polyurethane coating after storage in several different environmental conditions over the period of 14 days.

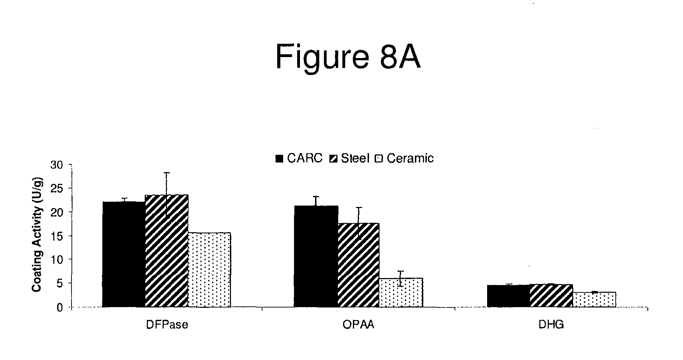

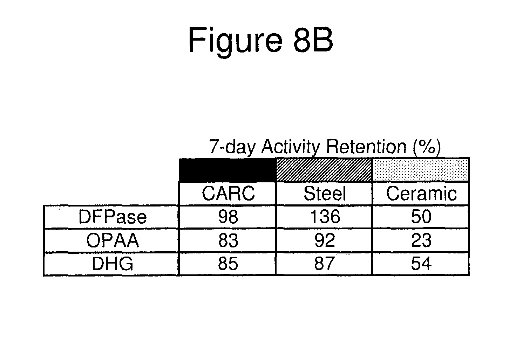

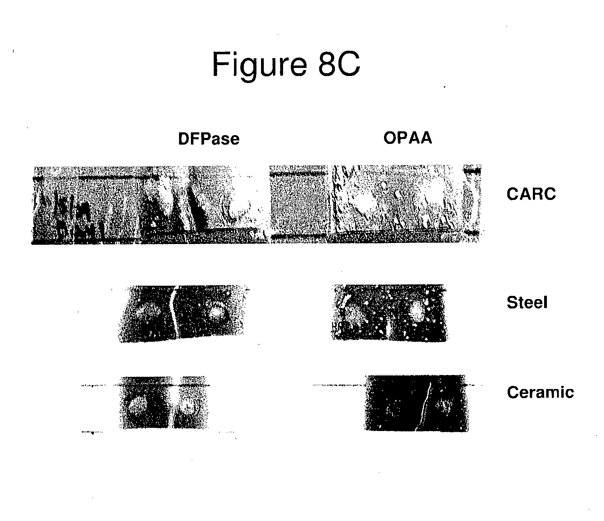

Figures 8A and 8B show the stability of the coating material of the present invention having either a DFPase, OPAA, or DHG containing enzyme as applied to CARC (Chemical Agent Resistant Coating), steel, or ceramic panels following a one week (7 days) exposure to the outdoor environment. Figure 8C shows the stability of the coating material of the present invention having either a DFPase or OPAA containing enzyme as applied to CARC, steel, or ceramic panels following a one week (7 days) exposure to the outdoor environment: left section--left droplet is detection of neat DFP by DFPase containing coating material of the present invention, and left section- right droplet is detection of dilute DFP by DFPase containing coating material of the present invention; right section-left droplet is detection of neat DFP by OPAA containing coating material of the present invention, and right section-right droplet is detection of dilute DFP by OPAA containing coating material of the present invention.

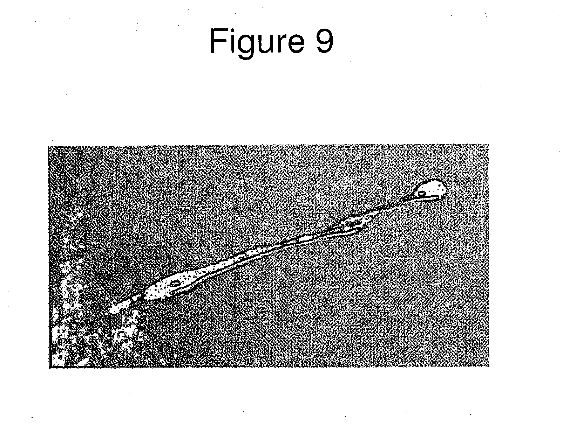

Figure 9 shows disclosure (detection) of a non-toxic colorless simulant by a coating material of the present invention that is used for training soldiers after the lapse of 5 minutes of time.

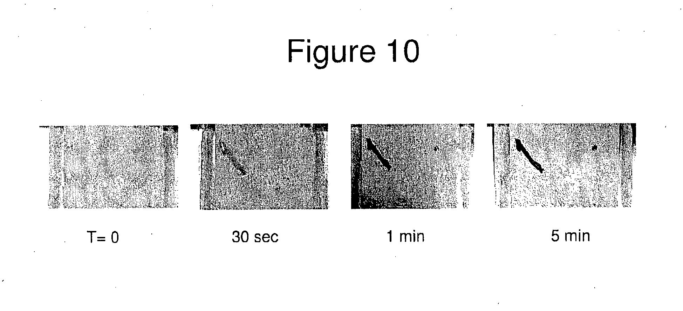

Figure 10 shows the coating material of the present invention subject to disclosure and detection testing with DFP, a G-series nerve agent simulant.

### Detailed Description of the Invention

While sorptive, strippable coatings can mitigate chemical hazards via sorption, they have no capability to actively detect or detoxify the chemical hazard. This invention combines the capability of sorptive strippable coatings that mitigate chemical agent hazards via sorption with biologically active enzymes capable of decontaminating and detoxifying chemical agents, namely G- and V-series organophosphate nerve agents and blister agents such as sulfur mustard, for the purpose of demonstrating a material that can absorb, decontaminate and disclose the presence of chemical warfare agents and/or other toxic materials. The decontamination enzymes in the formulation of the instant coating material include those which are used to deactivate or decontaminate a specific chemical hazard. The active enzyme can be changed relative to the need for detection or decontamination of a specific chemical hazard. The enzymes we use in our examples include, but are not limited to, diisopropylfluorophosphatase (DFPase), organophosphorus acid anhydrolase (OPAA), derivitized organophosphorus hydrolase (OPH) and dehalogenase (DHG). Detoxification of chemical agents, in particular G-series nerve agents and sulfur mustard (HD) produces strong acids. The concentration of strong acid decreases the local pH of the coating. In addition to the decontamination enzymes, the coating material contains reporter indicators which, preferably, are pH-sensitive dyes. The dyes employed in the examples set forth herein, include, but are not limited to, nitrazine yellow, thymol blue, bromothymol blue, phenol red, brilliant yellow and thymolphthalein. The dyes may be used alone, in combination, or as dye blends. The change in pH caused by agent hydrolysis drives the pH-sensitive dyes to undergo a chemical transition resulting in a colorimetric signal visible to an observer (using the naked eye) indicating the presence and physical location of contamination of toxic chemical. The strippable coatings of the present invention can be either water-borne or solvent-borne formulations that can cure rapidly (within 5 minutes and up to 48 hours) to a finish and are removable by employing mechanical means, such as for example, either peeling, use of water, or high pressure water rinse or a combination thereof. Chemistries for peelable coatings are diverse and can include, but are not limited to, the following polymers: polyurethane, polyacrylic, polyvinyl and polysiloxane. The enzyme containing coating functions as an absorbant of applied toxic chemicals preventing these hazards from breaking through to the underlying surface or from leaching back out of the coating. The enzymes within the coating hydrolyze and detoxify the applied toxic chemical burden (toxins or hazards), while through the use of additional indicating chemistry that comprises one or more reporter indicators such as for example one or more dyes or dye blends, and one or more buffers, trigger a localized color change at the toxic chemical contamination site. Any residual applied toxic chemicals (hazards) are eliminated when the coating material is removed (stripped) from the surface. The coating material of the present invention is stable enough to last from at least 1 week to more than several months in a field environment. Enzymes incorporated within the coating can detoxify the applied toxic chemicals and simulants using the humidity present in the air as the water required for the hydrolytic reaction. The disclosure chemistry (e.g., one or more dyes or optionally dye blends, and one or more buffers) incorporated within the coating can disclose to the visible eye the location of the applied toxic chemical(s) and simulant(s). As used herein, the terms toxic chemicals, chemical toxins, applied chemical agents, simulants, hazards, and contaminants may be used interchangeably.

The buffer solution of the present invention is critical to the coating material composition. One or more buffers are required in the coating material of the present invention for establishing and maintaining the pH of the coating in a pH range from about 5 to 10. The inclusion of at least one or more buffers in the coating material of the present invention is necessary to establish and maintain a suitable pH for enzyme activity as well as to mitigate false positives caused by benign interferents. These conditions, however, must be balanced against the requirement for disclosure sensitivity which is inhibited by excess buffering capacity in the coating. The hydrolytic enzyme(s) incorporated within the polymer coating(s) work most efficiently at neutral to slightly basic pH, but are much less active in acidic conditions. Buffering a coating provides an appropriate environment in which the enzyme can detoxify a contaminant even as the enzyme catalyzes hydrolytic reactions that generate acidic byproducts. Enzyme-containing coatings without buffer exhibit relatively poor decontamination efficacy because the acidity of the hydrolysis products caused a rapid drop in pH which subsequently deactivated the enzyme before it could further detoxify the toxic chemical or threat. Bis-Tris Propane (BTP) is a good buffer for this application as it has an effective buffering range from 6.3 to 9.5. Other appropriate buffer candidates and their useful pH ranges include, for example but not limited to, phosphate (pH 5.8-8.0), Trizma (pH 7.0-9.0) and borate (pH 7.4-9.2). Buffer is also crucial to establishing the original (non-contaminated) color of the coating. As the coating dries, it becomes more acidic. Without the buffer, a dye- and enzyme- containing coating may appear to possess the desired color as a liquid but would shift towards the acidic color as the coating cured. By adding a higher pH buffer (for example, BTP pH 9.0), it is possible to tune the color of the cured coating because the buffer reduces the acidification of the coating. This pH adjustment and control is necessary if the coating is to disclose the presence and location of contamination by exhibiting a color contrast between the clean and contaminated areas.

While previously developed peelable coatings are able to absorb a high degree of chemical agent contact, the use of enzymes to drive both decontamination and disclosure of chemical agent contamination is a revolutionary leap forward in peelable coatings defense technology. Furthermore, the strippable coating of the present invention is a passive sensing device, requiring minimal personnel involvement and thus reducing the logistical burden imposed by current 'active' detection technologies.

In the present invention, use of highly purified enzyme is preferred for immobilization within the coatings. The purity of the enzyme is important because increasing enzyme loading in coating formulations leads to improved detoxification and disclosure efficacy; however, the physical integrity of a coating is compromised when excessive protein is added. Enzyme concentrations up to 50 mg/ mL coating yield formulations that exhibit good physical properties such as coverage continuity, uniform texture, and reliable peelability. Extraneous protein content negatively impacts these characteristics. Background art US Patent Application No. 10/655,345 (McDaniel) describes the addition of a powderized bacterial cell pellet in which enzyme was produced to a coating formulation. This unpurified enzyme contains cell debris and extraneous protein which would limit loading and ultimately reduce the decontamination efficiency of the coating. As used herein, pure enzyme refers to enzyme that has undergone a process to remove cellular debris and extraneous protein which results in catalytically active enzyme. Purification of the enzyme prior to incorporation in the polymer coating maximizes the performance of the coating by ensuring that all the protein added is catalytically active.

The background art teaches coatings that require chemical means for the coating's removal from the underlying surface. The present invention comprises a temporary, peelable coating which can be removed by mechanical methods or forces (e.g. peeling by hand, or use of water). Residual coating can also be removed by power-washing. Harsh solvents and traditional paint strippers used to dissolve background art coatings are not necessary to accomplish removal of the coating of the present invention from a surface to which it is applied.

US Patent No. 6,759,220 B1 describes a method of immobilizing an enzyme within a polyurethane polymer foam. Enzymes incorporated within coatings represent an improvement over this background art in that an enzyme-containing coating can be applied to wide surface areas and would not be constrained to take the form of the container in which a foam is made. Furthermore, the current invention illustrates the incorporation of enzymes within coatings of various polymer compositions, broadening the application beyond polyurethane to include additional polymeric backbones (e.g. vinyl).

The present invention provides a coating material for absorbing a toxic chemical applied to a surface comprising at least one polymer coating, one or more purified enzymes immobilized or entrapped in the polymer coating for protecting a surface from contamination by one or more toxic chemicals, one or more reporter indicators that are responsive to one or more decontamination products of one or more toxic chemicals producing changes in pH for detecting and disclosing the location of one or more toxic chemicals, and one or more buffers for maintaining and establishing the pH of the coating between a pH ranging from 5 to 10, wherein the coating is removable from the surface by mechanical force applied to the surface. Preferably, the coating material comprises wherein the reporter indicator is one or more pH-responsive dyes or a blend of pH-responsive dyes. Preferably, the coating material comprises wherein the enzyme is selected from the group consisting of lyases, hydrolases, oxidoreductases, transferases, isomerases, and ligases, and combinations thereof. In general, six classes or types of enzymes (as classified by the type of reaction that is catalyzed) are recognized. Enzymes catalyzing reduction/oxidation or redox reactions are referred to generally as EC 1 (Enzyme Class 1) Oxidoreductases. Enzymes catalyzing the transfer of specific radicals or groups are referred to generally as EC2 (Enzyme Class 2) Transferases. Enzymes catalyzing hydrolysis are referred to generally asEC 3 Hydrolases. Enzymes catalyzing removal from or addition to a substrate of specific chemical groups are referred to generally as EC 4 Lyases. Enzymes catalyzing isomerization are referred to generally as EC 5 Isomerases. Enzymes catalyzing combination or binding together of substrate units are referred to generally as EC 6 Ligases. Hydrolase enzymes include, but are not limited to, a lipase, a phosphatase, an amylase, a cellulase, a protease, a peptidase, a urease, or a deaminase. Specific examples of suitable hydrolases include but are not limited to, organophosphorus hydrolase (OPH), organophosphorus acid anhydrolase (OPAA), urease, butyrylcholinesterase or acetylcholinesterase. One or a plurality of enzymes may be incorporated within the polymer coating. Thus, the coating material of the present invention comprises wherein one or more enzymes, or combinations thereof, decontaminate one or more toxic chemicals that can be a chemical warfare agent..

In a most preferred embodiment of the coating material of this invention as described herein, one or more purified enzymes are selected from the group consisting of one or more of organophosphorus acid anhydrolase (OPAA), organophosphorus hydrolase (OPH), dehalogenase (DHG), and diisopropylfluorophosphatase (DFPase).

In another embodiment of the coating material of the present invention as described herein, the polymer coating is selected from the group consisting of polyurethanes, polyacrylics, polysiloxanes, epoxies, polyacrylates, polyvinyls, polysilicone, fluorinated polymers, polypropylene, and polyethylene.

The coating material of the present invention absorbs fifty to one hundred percent of the toxic chemical upon contact with the coating within a period of from less than one minute to ninety minutes.

The coating material of the present invention is effective in disclosing, detecting, and detoxifying at least one or more of chemical warfare agents that are selected from the group consisting of V-series organophosphate nerve agents, G-series organophosphate nerve agents, non-traditional agents, sulfur mustard blister agents, and nitrogen mustard blister agents.

Another embodiment of the present invention provides a coating material as described herein comprising wherein the pH-responsive dye, dyes, or dye blend is colorimetric, fluorescent, or phosphorescent. Preferably, the coating material comprises wherein the pH-responsive dye, dyes, or dye blend is a colorimetric dye.

The coating material comprises wherein one or more enzymes hydrolyze the toxic chemical to produce acid hydrolysis products which cause the pH-responsive dye to change color visible to the naked eye. Preferably, the coating material as described herein contains one or more enzymes that are present in the amount of 1 to 12,000 Units per gram of the polymer coating. The enzymes are stable for periods of time ranging from at least one week and at least up to six months at ambient conditions. Most preferably, one or more of the enzymes are covalently immobilized or entrapped within the polymer coating. Optionally, one or more of the enzymes are modified employing known modification procedures with at least one or more polymer groups to enhance retention and stability of one or more enzymes within the polymer coatings. The polymer group are selected from the group consisting of poly(ethylene glycol), poly(vinyl alcohol), polyurethane, polyacrylic, polyvinyl, polysiloxane, fluorinated polymers, polypropylene, polyethylene, and combinations thereof.

In a preferred embodiment of this invention, the coating material, as described herein, includes wherein the individual component amounts per gram of dry cured polymer coating contains from about 3-600 micromole buffer, from about 1-12,000 Units purified enzyme, and from about 2-40 mg dye or dye blend.

The coating material of the present invention absorbs a toxic chemical and sequesters the toxic chemical from reaching the underlying surface to which the coating is applied. It will be understood that the coating material is applied to the clean surface prior to contamination with the toxic chemical.

In another embodiment of this invention, a method for absorbing, detoxifying, detecting, and disclosing the location of one or more toxic chemicals is provided, comprising applying a coating material to a surface wherein the coating material comprises at least one polymer coating, one or more purified enzymes immobilized or entrapped in the polymer coating, one or more reporter indicators that are responsive to one or more decontamination products of one or more toxic chemicals producing changes in pH, and one or more buffers for establishing and/or maintaining the pH of the coating ranging from a pH of about 5 to 10, wherein the coating is removable from the surface by mechanical force applied to the coating material, subjecting the coating material applied to the surface to at least one toxic chemical, and effecting absorption of one or more toxic chemicals by the coating material for detoxifying at least one or more of the toxic chemicals by one or more of the enzymes and for detecting and disclosing the location of one or more of the toxic chemicals by one or more of the reporter indicators. The method, as described herein, further includes removing the coating material containing the toxic chemical by mechanical force. The mechanical force is selected from the group of peeling the coating material by hand, by the use of water, or by the use of water under pressure.

The method, as described herein, further includes retaining the activity of one or more enzymes for a time period ranging from at least one week to at least six months.

In another embodiment of the present method, as described herein, the method further includes detoxifying a surface contaminated with levels of at least one toxic chemical ranging from 0.1 mg per square meter to 10 grams per square meter.

In a preferred embodiment of this invention, a coating material for absorbing a toxic chemical applied to a surface is provided comprising at least one polymer coating, one or more purified enzymes immobilized or entrapped in the polymer coating for protecting a surface from contamination by one or more toxic chemicals, one or more reporter indicators that are responsive to one or more decontamination products of one or more toxic chemicals producing changes in pH for detecting and disclosing the location of one or more toxic chemicals wherein a visual color change of the coating occurs and is observable to the naked eye, and one or more buffers for maintaining and/or establishing the pH of the coating between a pH from about 5 to 10, wherein the coating is removable from the surface by mechanical force applied to the surface. Preferably, the enzymes are covalently immobilized or entrapped within the polymer coating.

### Experimental Procedures

### 1. Disclosure of DFP, a G-series nerve agent simulant

Dye blends were developed for the coating materials of the present invention that would enable the coatings to exhibit relevant colors applicable for field military deployment. In one embodiment of the present invention, a green background color for the coating was utilized. Two pH-sensitive dyes were employed: nitrazine yellow and thymol blue. At slightly basic pH, these dyes were blue and yellow, respectively, and were combined (dye blend) to form a robust green color. The dye blend contained 0.75 mg/mL thymol blue and 0.25 mg/mL nitrazine yellow. The dye blend was incorporated in a water-borne polyurethane coating at a total concentration of 1 mg/mL. Buffer (150 mM Bis-Tris-Propane [BTP] pH 9.0) was included in the coating formulation to achieve the desired initial background color as well as to establish a favorable environment for the addition of immobilized enzyme, DFPase. Coatings containing the dye blend , buffer and 30 mg/mL DFPase were mixed, applied to CARC coated steel panels at 15 mil (375 µm) wet film thickness (wft), cured for 26 hours at 22-25% relative humidity (%RH) at 21-24 degrees centigrade (°C), and were subsequently exposed to neat diisopropylfluorophosphate (DFP) simulant. When microliter quantities of DFP were applied to the coated steel panels, the coating material of the present invention underwent a local color change as the enzyme catalyzed the hydrolysis of DFP thereby generating hydrofluoric acid. Nitrazine yellow transitioned from blue to yellow as pH dropped, causing a yellow streak to appear on the coating. As the pH dropped further, the thymol blue dye transitioned from yellow to red, causing an orange/red trace to form within the yellow streak on the coating. This coloration is indicative of the presence of a chemical agent simulant in sufficient quantity and can be seen in black and white in Figure 1. An initial color change can be detected visually within thirty seconds (30 seconds) and develops completely within five minutes and exhibits a strong signal that remains stable for several hours.

### 2. Decontamination of DFP, a G-series nerve agent simulant

This example sets forth the ability of the coating materials of the present invention to decontaminate G-series nerve agent and blister agent simulants. The methodology was based on Defence Standards issued by the UK Ministry of Defence. Defence Standard 00-72 defines the chemical agent resistance absorption and desorption requirement for coatings applied to military equipment. The test procedure was modified for use in the peelable coatings decontamination contact testing with agent simulant. Coatings were comprised of enzyme, indicator dyes and buffer. In this example of the invention, 30 mg/mL DFPase, 0.75 mg/mL thymol blue and 0.25 mg/mL nitrazine yellow as a dye blend, and 150 mM bis-tris propane (BTP) buffer at pH 9.0 were mixed with a water-borne 85% v/v polyurethane coating, applied at 15 mil wft (wet film thickness) to a 4" x 6" steel panel and cured for 16 hours at 19 °C and 45% RH. The coating panel was scored into 16 cm² sections and were contaminated with 5 g/m² DFP, a G-series nerve simulant, as neat droplets in a grid configuration. These tests were conducted in a chemical fume hood, enzyme-containing coatings were covered with a glass recrystallization dish after contamination to minimize simulant evaporation. Each section was analyzed separately for surface hazard, coating retention and breakthrough contamination immediately (t = 0 hours, n=2) or eight hours (n = 2) after contamination. Filter paper was used to blot surface simulant not absorbed by the coating. The peelable coating was then manually removed from the panel. Filter paper and the contaminated coating were extracted separately by iso-octane and analyzed by gas chromatography mass spectroscopy (GC-MS) using established methods well known by those persons skilled in the art. Diminishing simulant concentration as a function of time was used to quantify decontamination. Panels of coating containing either no protein or 30 mg/mL bovine serum albumin (BSA) instead of DFPase, but otherwise identical in composition to the aforementioned embodiment of the current invention were analyzed in parallel as negative controls. To demonstrate that the decontamination mechanism of response was enzyme-driven, a protein without enzyme activity (BSA) was added at the same concentration of the DFPase enzyme to a coating and subsequently analyzed. Very minimal impact on coating decontamination was observed in this control coating. Contamination concentration is reported in Figure 2 as a summation of filter paper contact and contaminated coating. No DFP was detected on the surface of coatings exposed to contamination after eight hours. The DFP was completely absorbed by the coating material of the present invention. No breakthrough to the underlying substrate panel was observed in any sample. The coating material of the present invention containing the immobilized pure enzyme, dye blend and buffer, decontaminated the simulant challenge, while the coatings containing proteins without enzymatic enzyme did not detoxify the DFP.

### 3. Enzyme-Loading Dependence of Decontamination

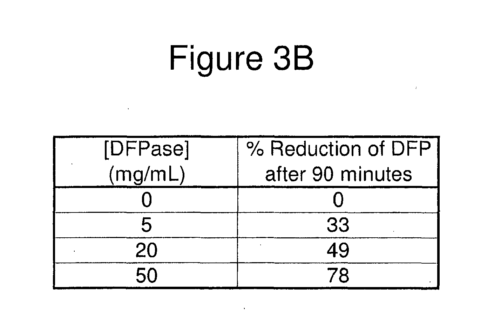

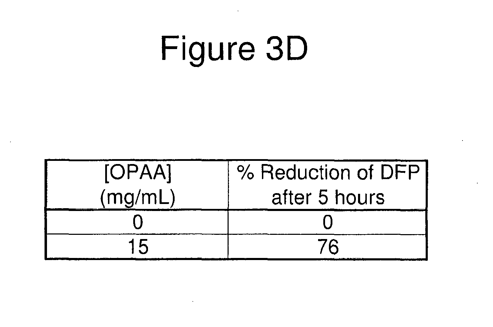

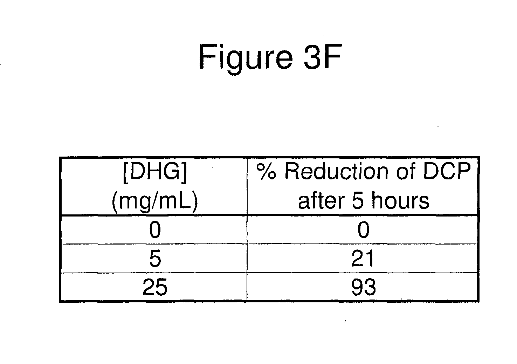

The purpose of this study was to demonstrate the effect of enzyme-loading on the decontamination efficacy of catalytic strippable coatings. The methodology was based on Defence Standards issued by the UK Ministry of Defence. Defence Standard 00-72 defines the chemical agent resistance absorption and desorption requirements for coatings applied to military equipment. The test procedure was modified for use in the peelable coatings decontamination contact testing with agent simulant. DFPase, OPAA and DHG were individually incorporated into water-borne polyurethane and polyacrylic peelable coatings at varying loading concentrations ranging from 5 to 50 mg enzyme/ mL coating with negative controls containing no enzyme also performed in parallel. Following the addition of buffered enzyme, the coatings were applied at 15 mil wet film thickness to steel panels and cured for 18 hours at ambient conditions. Coating panels were scored into 8 equal areas and each octant was contaminated with 5 g/m² neat simulant. DFP, a G-series nerve simulant, was applied to the DFPase and OPAA coatings while dichloropentane (DCP), an HD blister simulant, was utilized for the DHG coatings. As these tests were conducted in a chemical fume hood, coating panels were covered with a glass recrystallization dish following contamination to minimize simulant evaporation. Each section was analyzed separately for residual surface hazard, coating retention and breakthrough contamination at several time points ranging from fifteen minutes to five hours after contamination, in duplicate. Filter paper was used to blot surface simulant not absorbed by the coating. The peelable coating was then removed from the panel. Finally, any residual contaminant was collected from the underlying metal substrate. All three fractions were extracted by iso-octane and analyzed by GC-MS using established methods known by those persons skilled in the art. Diminishing simulant concentration as a function of time was used to quantify decontamination as shown in Figure 3. Contamination concentration is reported as a summation of contact and coating fractions. No breakthrough to the underlying substrate panel was observed in any sample.

Buffer (bis-tris propane (BTP) pH 9.0) was included in the formulation of these hydrolytic coatings to optimize performance. As DFPase concentration increased from 5 to 50 mg/mL coating, (Figure 3A-B) decontamination efficacy more than doubled (from 33% to 78%) after 90 minutes of hydrolysis. Decontamination efficiency improves as enzyme loading increases in enzyme concentration and extending contact time results in complete detoxification of the applied toxic chemical or simulant. A preferred embodiment of the current invention, 20 mg/mL DFPase coating exhibited good physical characteristics and hydrolyzed ~50% of the applied chemical simulant in 90 minutes. Longer time courses were studied for the OPAA (Figure 3C-D) and DHG (Figure 3E-F) coatings because their specific activities were lower than that of DFPase. The lower the specific activity of an enzyme, the slower it turns over substrate and thereby the longer it takes to decontaminate the applied chemical agent or simulant.

### 4. Buffer-Dependence of Decontamination

Increased buffer incorporation in coatings improves overall decontamination efficacy of chemical warfare agents and their simulants. In a preferred embodiment of this invention, the catalytic coating contains sufficient buffer to achieve detoxification of applied chemical agent (toxic chemical) while not so much buffer as to inhibit the pH-driven disclosure and detection capability. Enzyme-containing coatings without buffer resulted in poor decontamination efficacy because the acidity of the applied simulant and its hydrolysis products caused a drop in pH which subsequently deactivated the enzyme. The decontamination enzymes have an optimal pH in the neutral/ slightly basic range. In previously described embodiments of the invention, 150 mM BTP pH 9.0 buffer was utilized and shown both to detoxify and to disclose the location of applied simulant. In this example of the coating material of the current invention, a water-borne polyurethane coating containing 75 mM BTP pH 9.0 buffer and 30 mg/mL DFPase were prepared by mixing. This coating material was applied to bare metal panels at 15 mil wet film thickness (wft), and cured for 16 hours at 45% RH, 19 °C, and were subsequently challenged with 5 g/m² DFP. Residual contamination was analyzed by GC-MS following two, four or eight hours of exposure with the catalytic coating. Figure 4 shows that the coating containing 150 mM BTP buffer (ECC1) demonstrated better decontamination than an otherwise identical coating containing only 75 mM BTP buffer (ECC2). The coating with 150 mM BTP buffer detoxified 73% while its lower buffer counterpart detoxified 55% of the 5 g/m² DFP. Also included in the figure is a control coating containing 150 mM BTP buffer and no enzyme, analyzed in parallel, which did not show any appreciable decontamination of the applied simulant.

### 5. Disclosure of HD simulant by DHG coatings

In addition to detecting G-series nerve agent surrogates, an additional embodiment of the current invention demonstrates the capability to disclose the location of sulfur mustard (HD) simulant, dichloropentane (DCP). Polyurethane coatings containing 10 mg DHG/ mL coating, 15 mM BTP pH 9.0 buffer and 1 mg dye (75% thymol blue; 25% nitrazine yellow) / mL coating were applied to three substrate materials (steel, polyurethane chemical agent resistant coating (CARC), and ceramic) and subsequently challenged with neat and dilute DCP. Following a 24-hour cure, 2 µL neat or 4 µL dilute (50% v/v water) droplets of DCP were spotted onto the catalytic coating. As shown in Figure 5A, water-borne polyurethane material coating of this invention disclosed the location of the dilute DCP within 30 minutes of contamination by transitioning from green to yellow (shown in black and white in Figure 5A). Disclosure is enhanced in the dilute sample because additional water increases the catalytic rate of DHG, increasing the speed of DCP hydrolysis. The hydrolysis generates acid, which decreases the pH in the immediate vicinity of the contaminant and in turn drives the colorimetric shift of the dyed coating. No color change was observed when DCP (neat or dilute) was applied to a control coating containing dyes but no enzyme. In a more preferred embodiment of the invention, buffer concentration was reduced to improve disclosure of DCP by a catalytic coating. A water-borne vinyl coating containing 10 mg DHG /mL coating, 1.5 mM BTP pH 9.0 buffer and 1 mg dye (75% thymol blue; 25% nitrazine yellow) / mL coating was applied to a ceramic tile at 30 mil wft and allowed to cure for 24 hours before being challenged with neat and dilute DCP as described above. The reduction in buffer results in a faster, more visible disclosure of DCP because fewer acid byproducts of catalysis are needed to drive the colorimetric change in the coating. Figure 5B illustrates these results in black and white; dilute DCP (right spot) yields a more visible color change than the neat contaminant (left spot) due to the presence of water to facilitate hydrolysis.

### 6. Dual Enzyme Coatings

Following earlier efforts to generate a relevant color scheme for enzyme containing coatings for military use, a multi-enzyme coating for broad-spectrum disclosure and decontamination was developed. Coatings were formulated with either green or tan dye chemistries and included two hydrolases within a single coating. When agent simulant was applied, the coating locally changed color as the enzyme turned over simulant and generated strong acids. In this example, water-borne polyurethane and vinyl coatings each comprising 5 mg DFPase, 5mg DHG, 0.75 mg thymol blue and 0.25 mg nitrazine yellow per mL coating, with either 1.5 or 15 mM BTP pH 9.0 buffer, were applied to ceramic tiles at 30 mil wft and allowed to cure for 16 hours. Subsequent application of neat and dilute simulant yielded disclosure of DFP on all of the coating samples in less than ten minutes. A clearly visible yellow-orange spot developed on the green coating over a 60 minute time-lapse in the location of the applied G-series nerve simulant as seen in the top row of each of the four coating squares in Figure 6A (shown in black and white). Dilute DCP also prompted a color change, especially noticeable in the vinyl coating samples of Figure 6A (lower right quadrant of each coating square), demonstrating that an HD simulant can also be detected in the dual-enzyme broad-spectrum catalytic coating. While the color development was slower in this particular example compared to that of single-enzyme coatings, positive disclosure of both G-series nerve agents and sulfur mustard analogs within a single protective, peelable, catalytic coating material of this invention is demonstrated.

A more preferred embodiment of the invention manifests broad-spectrum disclosure in a more analogous intensity. That is, similar amounts of applied contaminant (whether nerve or blister agents) should yield a relatively equivalent color change response. By reducing the concentration of DFPase and increasing that of DHG, keeping all other coating material elements the same, enhanced sensitivity to DCP was achieved while also maintaining excellent disclosure of DFP. In this preferred embodiment of the current invention, vinyl peelable coating material was prepared containing 10 mg DHG, 2 mg DFPase, 7.5 mg thymol blue, and 2.5 mg nitrazine yellow per mL coating and 1.5 mM BTP pH 9.0 and applied at 30 mil wft to a ceramic tile. As shown in Figure 6B, the coating was challenged with either neat (left) or dilute (right) DFP (top) or DCP (bottom) and the color development was allowed to progress for 60 minutes. DFP disclosure is strong and rapid, as illustrated by the yellow/red color change in both the neat and dilute contamination spots. DCP disclosure is less intense; however, the dilute application yielded a more visible shift to yellow against the green coating than when only 5 mg DHG / mL coating was tested (shown in black and white in Figure 6B).

Individual enzyme activity was not significantly reduced by the inclusion of both hydrolases in a single coating. That is, neither enzyme inhibited the activity of the other. In previous studies, an inverse relationship between buffer strength and disclosure sensitivity in enzyme-containing coatings was observed. Since the addition of two enzymes increases the putative buffering capacity of the coating, it is plausible that inhibition of disclosure would occur because the dyes do not undergo a sufficient pH-dependent color change. Further purification of the enzymes prior to inclusion in the coating will further improve the sensitivity of disclosure.

### 7. Operational Stability of Coatings: Single Variable Environmental Testing

Stability studies were conducted to determine the operational stability of the catalytic coatings, specifically enzyme retention thereof. A preferred embodiment of the current invention will withstand, at minimum, a week-long exposure to several environmental conditions. Each of three buffered hydrolases (DFPase, OPAA or DHG) at 5 mg enzyme/mL coating were incorporated into a water-borne polyurethane or vinyl coating. Enzyme-containing coatings were applied to a CARC panel at 30 mil wft and allowed to cure overnight at ambient conditions before testing. Three environmental variables were studied in this test: humidity, temperature and light exposure. Samples were subjected to one of the following seven conditions for two weeks: 4 °C, 20 °C, 40 °C, 15% relative humidity (RH), 95% RH, constant fluorescent light, and constant darkness. Samples were taken before exposure (Day 0) and following 1, 4, 7, and 14 days at each condition. Enzyme activity was determined by measuring the rate of evolution of enzymatic hydrolysis products in a stirred reactor vial containing the enzyme substrate and a 4 cm² section of catalytic coating using an ion-specific electrode. DFPase and OPAA catalyze the hydrolysis of diisopropylfluorophosphate (DFP), a G-series nerve simulant, which generates fluoride ions monitored by a fluoride ion-specific electrode. DHG catalyzes the hydrolysis of dibromoethane, an HD simulant, which generates bromide ions monitored by a bromide ion-specific electrode. Figures 7A and 7B show a preferred embodiment of the current invention in which a DFPase coating retains >60% immobilized catalytic activity for two weeks in six of seven environmental conditions tested. The current invention also exhibits excellent physical characteristics (adhesion, peelability and continuity of coverage) after environmental exposure. Similar trends in catalytic activity and material properties were observed in OPAA- and DHG- containing coatings as shown in Figures 7C and 7D, and 7E and 7F, respectively.

### 8. Complete Coating: Outdoor Exposure Testing

This study was designed to assess physical/chemical properties, decontamination efficacy and disclosure capability of catalytic coatings on multiple surfaces during a simulated field operation. A preferred embodiment of the current invention is capable of decontamination and disclosure of chemical warfare agents and their simulants following exposure to the outdoor environment for an extended period of time. Sample coatings were prepared by dissolving one hydrolytic enzyme (DFPase, OPAA or DHG) and dye powder (thymol blue and nitrazine yellow) in buffer (100 mM BTP, pH 9.0) and mixing the enzyme and dye solution with a clear water-borne polyurethane peelable coating to make a final concentration of 10 mg enzyme/mL coating, 1 mg dye/ mL coating, 15 mM buffer. Enzyme-containing coatings were applied in 2 cm strips at 30 mil wet film thickness to the following substrate panels: bare steel, CARC-coated steel, and ceramic. Samples were allowed to cure overnight for approximately 16 hours at ambient room temperature and humidity conditions before testing. Temperature ranged from -1.5 to 23 °C and relative humidity fluctuated between 18 and 89% over the week-long outdoor study. Decontamination enzyme activity retention was assayed by measuring the rate of evolution of enzymatic hydrolysis products in a stirred reactor vial containing the enzyme substrate and a 4 cm² section of catalytic coating using an ion-specific electrode. DFPase and OPAA catalyze the hydrolysis of diisopropylfluorophosphate (DFP), a G-series nerve simulant, which generates fluoride ions monitored by a fluoride ion-specific electrode. DHG catalyzes the hydrolysis of dibromoethane, an HD simulant, which generates bromide ions monitored by a bromide ion-specific electrode. Enzyme activity retention on each substrate material is summarized in Figures 8A and 8B. All DFPase and DHG retained >50% of initial coating activity following one week exposure to the outdoor environment. OPAA coatings applied to CARC and steel panels exceeded these performance specifications as well. Physical adhesion and peelability were assessed qualitatively for each sample section prior to use in the enzyme activity assay. Each embodiment of the present invention described herein exhibited good physical characteristics at all time points analyzed. Disclosure efficacy was determined by spotting neat or dilute simulant (DFP for DFPase and OPAA coatings; DCP for DHG coatings) and observing color change from green to yellow or red in the coating. Disclosure samples are shown in Figure 8C (in black and white). DFPase and OPAA coatings disclosed applied simulant contamination within 30 minutes by undergoing a distinct color change from green to yellow/orange. DHG coatings also disclose the presence of contamination; however the rate and intensity of color change is less dramatic due to lower activity of DHG. Following disclosure, visual inspection of the coating and underlying substrate was utilized to ascertain the ability of these coatings to resist breakthrough of applied chemical simulant.

### 9. Training Coating

A training version of the chemical warfare agent disclosure/decontaminant coating was developed. This embodiment of the current invention has utility for demonstrations in which live hazardous agent or harsh pesticide simulant contamination is not possible or desired. This training coating uses a different enzyme, urease, and a benign chemical simulant, urea, to mimic the color change response of the live-agent version of the coating. A tan water-borne vinyl coating containing 15 mg urease, 80 mg phenol red per mL coating and 45 mM potassium phosphate pH 7.0 buffer was mixed and applied to bare metal panels and a CARC-coated vehicle tailgate at 20 mil wft. Following a 24 hour cure at ambient room conditions, a clear top-coat containing 25 mM potassium phosphate pH 7.0 buffer was applied at 10 mil wft and allowed to cure an additional 24 hours. Once applied to a surface and given time to cure, the training coating detects and discloses the location of applied simulant via a color change within ten minutes. The training version of the coating is tan and, in the presence of non-toxic urea based simulant, turns red. Color development was monitored over time and is illustrated in Figure 9 (shown in black and white). The coatings responded with a visible color change in approximately ten minutes. After disclosure, the coating was removed by scoring an edge and peeling the majority of the coating from the underlying surface. Residual coating may be removed using a high pressure wash. The coating inhibits any contamination breakthrough and thus protects the surface of the underlying object.

### 10. Peelable Coating

This example of the coating material of the present invention was prepared as follows: thymol blue dye powder was dissolved in 100 mM BTP pH 9.0 buffer to a concentration of 66.7 mg/mL. This represents a 10-fold increase in dye content as in previous examples of the coatings of this invention. A 900uL aliquot of the dye solution was used to dissolve 180 mg DFPase. This dye and enzyme solution was added to 5.1 mL tan colored polyurethane coating. Final component concentrations were: 30 mg/mL DFPase, 10 mg/mL thymol blue, 15 mM BTP pH 9.0. The polymer coating, enzyme, dye and buffer containing coating material was mixed vigorously for approximately five minutes to ensure homogeneity and subsequently applied to steel panels at 15 mil wet film thickness (wft). The coatings were allowed to cure at ambient lab conditions (22+/- 1 °C, 26 +/- 1 % RH) overnight for approximately 18 hours. The cured coating material applied to the steel panels were tan in color.

The coating material was then subjected to disclosure (detection) testing. Neat DFP was used as a G-series nerve agent simulant. 8 uL neat DFP was applied in a diagonal streak to the upper left corner of the coated panel, representing a 5 g/m² DFP challenge to a 16 cm² section. 1 uL neat DFP was applied in a single droplet to the upper left corner of the coated panel, representing a 0.625 g/m² DFP challenge to the 16 cm² section. The coating underwent a localized color change from tan to red indicating the presence and location of both areas of applied contamination of DFP. This color change was caused by the transition of the pH-sensitive thymol blue dye from yellow to red driven by the acid generated during the DFPase-catalyzed hydrolysis of DFP. Color development in the coating was visible within 60 seconds and reached its maximum contrast in less than ten minutes. Time lapse photographs after 5 minutes document the DFP disclosure and detection by the coating material in Figure 10 (shown in black and white).

## Claims

1. A coating material for absorbing a toxic chemical applied to a surface comprising:
at least one polymer coating;
one or more purified enzymes immobilized or entrapped in said polymer coating for protecting a surface from contamination by one or more toxic chemicals;
one or more reporter indicators that are responsive to one or more decontamination products of said one or more toxic chemicals producing changes in pH for detecting and disclosing the location of said one or more toxic chemicals; and
one or more buffers for establishing and/or maintaining the pH of said coating between a pH ranging from about 5 to 10, wherein said coating is removable from the surface by mechanical force applied to the surface.

2. The coating material of Claim 1 comprising wherein said reporter indicator is one or more pH-responsive dyes or a blend of pH-responsive dyes, and wherein said enzyme is selected from the group consisting of lyases, hydrolases, oxidoreductases, transferases, isomerases, and ligases, and combinations thereof.

3. The coating material of Claim 1 comprising wherein said one or more enzymes, or combinations thereof, decontaminate said one or more toxic chemicals that is/are a chemical warfare agent/agents.

4. The coating material of Claim 1 comprising wherein said one or more purified enzymes are selected from the group consisting of one or more of organophosphorus acid anhydrolase (OPAA), organophosphorus hydrolase (OPH), dehalogenase (DHG), and diisopropylfluorophosphatase (DFPase).

5. The coating material of Claim 1 comprising wherein said polymer coating is selected from the group consisting of polyurethanes, polyacrylics, polysiloxanes, epoxies, polyacrylates, polyvinyls, polysilicone, fluorinated polymers, polypropylene, and polyethylene.

6. The coating material of Claim 2 comprising wherein said pH-responsive dye, dyes, or dye blend is colorimetric, fluorescent, or phosphorescent.

7. The coating material of Claim 1 comprising wherein said one or more of said enzymes are modified with at least one or more polymer groups to enhance retention and stability of said one or more enzymes within said polymer coatings.

8. A method for absorbing, detoxifying, detecting, and disclosing the location of one or more toxic chemicals comprising:
applying a coating material to a surface wherein said coating material comprises at least one polymer coating, one or more purified enzymes immobilized or entrapped in said polymer coating, one or more reporter indicators that are responsive to one or more decontamination products of said one or more toxic chemicals producing changes in pH, and one or more buffers for establishing and/or maintaining the pH of said coating ranging from a pH of about 5 to 10, wherein said coating is removable from the surface by mechanical force applied to the coating material;
subjecting said coating material applied to said surface to at least one toxic chemical; and
effecting absorption of said one or more toxic chemicals by said coating material for detoxifying at least one or more of said toxic chemicals by one or more of said enzymes and for detecting and disclosing the location of one or more of said toxic chemicals by one or more of said reporter indicators.

9. The method of Claim 8 comprising removing said coating material containing said chemical toxin by mechanical force.

10. The method of Claim 8 comprising including retaining the activity of said one or more enzymes for a time period ranging from at least one week to at least six months.

## Patentansprüche

1. Ein Beschichtungsmaterial zum Absorbieren einer toxischen Chemikalie, welches auf einer Oberfläche aufgebracht ist, das umfasst:
mindestens eine Polymerbeschichtung;
ein oder mehrere gereinigte(s) Enzym(e), die auf der Polymerbeschichtung immobilisiert sind oder in ihr eingefangen sind, zum Schutz der Oberfläche vor Verunreinigung durch eine oder mehrere toxische Chemikalie(n) ;
ein oder mehrere Reporterindikatoren, die auf ein oder mehrere Dekontaminationsprodukt(e) von der einen oder den mehreren toxischen Chemikalie(n) ansprechbar sind, wodurch Änderungen des pH-Werts erzeugt werden, zum Nachweis oder zur Offenbarung der Lokalisierung der einen oder der mehreren toxischen Chemikalie(n) ; und
ein oder mehrere Puffer zum Einstellen und/oder Aufrechterhalten des pH-Werts der Beschichtung im Bereich von etwa 5 bis 10, wobei die Beschichtung von der Oberfläche durch mechanische Kraft, die auf die Oberfläche angewendet wird, entfernbar ist.

2. Das Beschichtungsmaterial nach Anspruch 1, umfassend wobei der Reporterindikator ein oder mehrere auf den pH-Wert ansprechbare Farbstoffe oder eine Mischung der auf den pH-Wert ansprechbaren Farbstoffe sind, und wobei das Enzym ausgewählt ist aus der Gruppe bestehend aus Lyasen, Hydrolasen, Oxidoreduktasen, Transferasen, Isomerasen und Ligasen, und Kombinationen davon.

3. Das Beschichtungsmaterial nach Anspruch 1, umfassend wobei das eine oder mehrere Enzym(e), oder Kombinationen davon, das eine oder die mehreren toxische(n) Chemikalie(n) dekontaminiert, welche(s) (ein) chemische(r) Kampfstoff(e) ist/sind.

4. Das Beschichtungsmaterial nach Anspruch 1, umfassend wobei das eine oder die mehreren gereinigte(n) Enzym(e) ausgewählt sind aus der Gruppe bestehend aus einem oder mehreren von Organophosphorsäueanhydrolase (OPAA), Organophosphorhydrolase (OPH), Dehalogenase (DHG) und Düsopropylfluorphosphatase (DFPase).

5. Das Beschichtungsmaterial nach Anspruch 1, umfassend wobei die Polymerbeschichtung ausgewählt ist aus der Gruppe bestehend aus Polyurethanen, Polyacrylen, Polysiloxanen, Epoxiden, Polyacrylaten, Polyvinylen, Polysilikonen, fluorierten Polymeren, Polypropylen und Polyethylen.

6. Das Beschichtungsmaterial nach Anspruch 2, umfassend wobei der/die auf den pH-Wert ansprechbare(n) Farbstoff, Farbstoffe oder Farbstöffmischung kolorimetrisch, fluoreszierend oder phosphoreszierend ist/sind.

7. Das Beschichtungsmaterial nach Anspruch 1, umfassend wobei das eine oder die mehreren Enzym(e) mit mindestens einer oder mehreren polymeren Gruppen modifiziert sind um die Retention und Stabilität des einen oder der mehreren Enzym(s)(e) innerhalb der Polymerbeschichtungen zu verbessern.

8. Ein Verfahren zum Absorbieren, Entgiften, Nachweisen und Offenbaren der Lokalisierung der einen oder der mehreren toxischen chemische(n) Chemikalie(n) umfassend:
Aufbringen eines Beschichtungsmaterials auf die Oberfläche, wobei das Beschichtungsmaterial mindestens eine Polymerbeschichtung, ein oder mehrere gereinigte(s) Enzym(e), die auf der Polymerbeschichtung immobilisiert sind oder darin eingefangen sind, ein oder mehrere Reporterindikator(en), die auf ein oder mehrere Dekontaminationsprodukt(e) des einen oder der mehreren toxischen Chemikalien ansprechbar sind, wobei Änderungen des pH-Werts erzeugt werden, und einen oder mehrere Puffer zum Einstellen und/oder Aufrechterhalten des a pH-Werts der Beschichtung im Bereich von etwa 5 bis 10, umfasst, wobei die Beschichtung von der Oberfläche durch mechanische Kraft, die auf die Oberfläche angewendet wird, entfernbar ist;
Unterwerfen des Beschichtungsmaterials, das auf die Oberfläche aufgebracht wurde mindestens einer toxischen Chemikalie; und
Bewirken der Absorption der einen oder der mehreren toxischen Chemikalie(n) durch das Beschichtungsmaterial zum Entgiften von mindestens einer oder von mehreren toxischen Chemikalie durch ein oder mehrere Enzym(e) zum Nachweis oder der Offenbarung der Lokalisierung der einen oder der mehreren toxischen Chemikalie(n) durch einen oder mehrere Reporterindikator(en).

9. Das Verfahren nach Anspruch 8, umfassend dem Entfernen des Beschichtungsmaterials, welches das chemische Toxin enthält, durch mechanische Kraft.

10. Das Verfahren nach Anspruch 8, umfassend einschließlich der Beibehaltung der Aktivität des einen oder der mehreren Enzyme für einen Zeitraum von mindestens einer Woche bis mindestens sechs Monaten.

## Revendications

1. Matériau de revêtement permettant d'absorber un produit chimique toxique appliqué à une surface comprenant:
au moins un revêtement polymère:
un ou plusieurs enzymes purifiés immobilisés ou piégés dans ledit revêtement polymère permettant de protéger une surface de toute contamination par un ou plusieurs produits chimiques toxiques;
un ou plusieurs indicateurs reporteurs réagissant à un ou plusieurs produits de décontamination desdits un ou plusieurs produits chimiques toxiques produisant des changements de pH pour détecter et divulguer l'emplacement desdits un ou plusieurs produits chimiques toxiques; et
un ou plusieurs tampons permettant d'établir et/ou de maintenir le pH dudit revêtement entre un pH de 5 et un pH de 10 environ, où ledit revêtement peut s'enlever de la surface en appliquant une force mécanique à la surface.

2. Matériau de revêtement selon la revendication 1, où ledit indicateur reporteur correspond à un ou plusieurs colorants sensibles au pH ou un mélange de colorants sensibles au pH, ou où ledit enzyme est sélectionné parmi le groupe consistant en lyases, hydrolases, oxidoréductases, transférases, isomérases et ligases, et leurs combinaisons.

3. Matériau de revêtement selon la revendication 1, où lesdits un ou plusieurs enzymes ou des combinaisons de ceux-ci décontaminent lesdits un ou plusieurs produits chimiques toxiques constituant un ou des agents de guerre chimique.

4. Matériau de revêtement selon la revendication 1, où lesdits un ou plusieurs enzymes purifiés sont sélectionnés parmi le groupe consistant en un ou plusieurs de l'anhydrolase d'acide organophosphoré (AAOP), d'hydrolase organophosphoré (HOP), de déhalogénase (DHG) et diisopropylfluorophosphatase (DFPase).

5. Matériau de revêtement selon la revendication 1, où ledit revêtement polymère est anneau sélectionné parmi le groupe consistant en polyuréthanes, polyacryliques, polysiloxanes, époxies, polyacrylates, polyvinyles, polysilicone, polymères fluorés, polypropylène et polyéthylène.

6. Matériau de revêtement selon la revendication 2, où lesdits colorant, colorants ou mélange de colorants sensibles au pH sont colorimétriques, fluorescents ou phosphorescents.

7. Matériau de revêtement selon la revendication 1, où lesdits un ou plusieurs desdits enzymes sont modifiés par au moins un ou plusieurs groupes polymères pour renforcer la rétention et la stabilité desdits un ou plusieurs enzymes dans lesdits revêtements polymères.

8. Procédé d'abosrption, de détoxication, de détection et de divulgation de l'emplacement d'un ou plusieurs produits chimiques toxiques comprenant:
l'application d'un matériau de revêtement à une surface où ledit matériau de revêtement contient au moins un revêtement polymère, un ou plusieurs enzymes purifiés immobilisés ou piégés dans ledit revêtement polymère, un ou plusieurs indicateurs reporteurs sensibles à un ou plusieurs produits de décontamination desdits un ou plusieurs produits chimiques toxiques produisant des changements de pH, et un ou plusieurs tampons permettant d'établir et/ou de maintenir le pH dudit revêtement entre un pH de 5 et un pH de 10 environ, où ledit revêtement peut s'enlever de la surface en appliquant une force mécanique au matériau de revêtement;
l'application dudit matériau de revêtement présentant sur ladite surface sur au moins un produit chimique toxique; et
l'absorption desdits un ou plusieurs produits chimiques toxiques par ledit matériau de revêtement pour détoxiquer au moins un ou plusieurs desdits produits chimiques toxiques d'ici par un ou plusieurs desdits enzymes et pour détecter et divulguer l'emplacement d'un ou plusieurs desdits produits chimiques toxiques à l'aide d'un ou plusieurs desdits indicateurs reporteurs.

9. Procédé selon la revendication 8, comprenant l'élimination dudit matériau de revêtement contenant ladite toxine chimique en appliquant une force mécanique.

10. Procédé selon la revendication 8 comprenant la rétention de l'activité desdits un ou plusieurs enzymes pendant une période allant d'au moins une semaine à au moins six mois.
